(19) Europäisches Patentamt
European Patent Office
Office européen des brevets

(11) EP 3 846 927 B1

(12) EUROPÄISCHE PATENTSCHRIFT

(45) Veröffentlichungstag und Bekanntmachung des Hinweises auf die Patenterteilung:
**23.11.2022 Patentblatt 2022/47**

(21) Anmeldenummer: **19762155.0**

(22) Anmeldetag: **03.09.2019**

(51) Internationale Patentklassifikation (IPC):
***B01J 3/04*** (2006.01) ***B01J 10/00*** (2006.01)
***B01J 19/00*** (2006.01) ***B01J 19/24*** (2006.01)
***B01J 19/26*** (2006.01) ***C07B 53/00*** (2006.01)
***C07B 57/00*** (2006.01) ***C07C 45/50*** (2006.01)
***B01J 31/24*** (2006.01) ***B01J 31/28*** (2006.01)

(52) Gemeinsame Patentklassifikation (CPC):
(C-Sets verfügbar)
**B01J 3/042; B01J 10/00; B01J 19/006; B01J 19/2435; B01J 19/244; B01J 19/26; C07B 53/00; C07C 29/172; C07C 29/56; C07C 45/62;** B01J 31/24; B01J 31/2409; B01J 2219/00768; B01J 2219/00777; B01J 2219/0871; (Forts.)

(86) Internationale Anmeldenummer:
**PCT/EP2019/073472**

(87) Internationale Veröffentlichungsnummer:
**WO 2020/048986 (12.03.2020 Gazette 2020/11)**

(54) **REAKTOR ZUR DURCHFÜHRUNG EINER REAKTION ZWISCHEN ZWEI NICHT MISCHBAREN FLUIDEN UNTERSCHIEDLICHER DICHTE**

REACTOR FOR CARRYING OUT A REACTION BETWEEN TWO IMMISCIBLE FLUIDS OF DIFFERENT DENSITIES

RÉACTEUR DESTINÉ À LA MISE EN OEUVRE D’UNE RÉACTION ENTRE DEUX FLUIDES NON MISCIBLES DE DENSITÉ DIFFÉRENTE

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **05.09.2018 EP 18192755**

(43) Veröffentlichungstag der Anmeldung:
**14.07.2021 Patentblatt 2021/28**

(73) Patentinhaber: **BASF SE**
**67056 Ludwigshafen am Rhein (DE)**

(72) Erfinder:
- **BEY, Oliver**
  **67056 Ludwigshafen (DE)**
- **ZEHNER, Peter**
  **67273 Weisenheim am Berg (DE)**
- **SCHELWIES, Mathias**
  **67056 Ludwigshafen (DE)**
- **PACIELLO, Rocco**
  **67056 Ludwigshafen (DE)**
- **HAUBNER, Martin**
  **67056 Ludwigshafen (DE)**
- **WEGNER, Guenter**
  **67354 Roemerberg (DE)**
- **TEBBEN, Gerd**
  **67056 Ludwigshafen (DE)**
- **HEYDRICH, Gunnar**
  **67056 Ludwigshafen (DE)**
- **SEEBER, Georg**
  **67056 Ludwigshafen (DE)**
- **ACKER, Michael**
  **67056 Ludwigshafen (DE)**

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).

EP 3 846 927 B1

(74) Vertreter: **BASF IP Association**
**BASF SE**
**GBI-C006**
**67056 Ludwigshafen (DE)**

(56) Entgegenhaltungen:
**WO-A1-2016/097242** **DE-A1-102004 003 468**
**US-A- 4 000 212**

(52) Gemeinsame Patentklassifikation (CPC): (Forts.)
B01J 2219/0884; B01J 2219/185; B01J 2219/1943; B01J 2231/643; B01J 2531/822

C-Sets
**C07C 29/172, C07C 35/12;**
**C07C 29/56, C07C 35/17;**
**C07C 45/62, C07C 47/21**

## Beschreibung

Die vorliegende Erfindung betrifft einen Reaktor zur Durchführung einer Reaktion zwischen zwei nicht mischbaren Fluiden unterschiedlicher Dichte und ein Verfahren zur Durchführung einer kontinuierlichen Reaktion in einem solchen Reaktor.

Zahlreiche Umsetzungen werden unter Inkontaktbringen von nicht mischbaren Fluiden unterschiedlicher Dichte durchgeführt. Beispielsweise bedient sich die Hydrierung organischer Verbindungen der Umsetzung einer gelösten hydrierbaren Verbindung mit gasförmigen Wasserstoff. Es ist bekannt, die Hydrierung in einer ersten rückvermischten Zone und einer zweiten Zone mit begrenzter Rückvermischung durchzuführen. Die Rückvermischung in der ersten Zone erlaubt eine zweckmäßige Abfuhr der Reaktionswärme, während die zweite Zone der Vervollständigung des Reaktionsumsatzes dient.

Die WO 2009/153123 beschreibt ein kontinuierliches Verfahren zur Hydrierung organischer Verbindungen in einem mehrphasigen System in Gegenwart eines homogenen oder heterogenen Katalysators, wobei man das Verfahren in zwei Stufen durchführt, wobei die erste Stufe in einem Schlaufenreaktor mit externem Wärmetauscher und die zweite Stufe in einem Blasensäulenreaktor mit begrenzter Rückvermischung durchgeführt wird.

Die EP 1 338 333 A1 beschreibt eine Reaktorkaskade aus geschlossenem Haupt- und geschlossenem Nachreaktor, wobei der Nachreaktor sich im Innern des Hauptreaktorbehälters befindet.

Die EP 1 231 198 A1 beschreibt ein kontinuierliches Verfahren zur Hydroformylierung von Olefinen, bei denen ein zwei nacheinander durchströmbare Reaktionsräume umfassender Reaktor verwendet wird, wobei der zweite Reaktionsraum Lochbleche aufweisen kann.

Die DE 10 2004 003468 A1 beschreibt eine Vorrichtung zum mikrobiologischen Abbau von Schadstoffen in Fluiden mit mindestens zwei Aufnahmreäumen für das Fluid. Die US 4,000,212 A beschreibt ein Verfahren zur Alkylierung eines Kohlenwasserstoffs in einem in mehrere Zonen unterteilten Reaktor. Die WO 2016/097242 A1 beschreibt ein Verfahren zur Beeinflussung der Schaumbildung bei Gas-Fllüssigkeits-Reaktionen in Begasungsreaktoren.

Zwar ist eine ausreichende Dispergierung des gasförmigen Reaktionspartners Wasserstoff im flüssigen Reaktionsgemisch für den Reaktionsfortgang erforderlich. Andererseits erfolgt die Hydrierung selbst in der flüssigen Phase. Die Anwesenheit von Wasserstoffgas über die für den Hydrierumsatz notwendige Menge hinaus mindert daher das für die Hydrierung zur Verfügung stehende Reaktionsvolumen. Es ist vorteilhaft, den zur Verfügung stehenden Reaktionsraum optimal zu nutzen.

Eine derartige Problematik besteht grundsätzlich bei der Reaktion von nicht miteinander mischbaren Fluiden unterschiedlicher Dichte. Der Erfindung liegt die Aufgabe zugrunde, einen Reaktor zur Durchführung einer Reaktion zwischen zwei nicht mischbaren Fluiden unterschiedlicher Dichte bereitzustellen, der eine optimale Verweilzeitverteilung gemäß den Anforderungen der jeweiligen Reaktion erlaubt.

Die Aufgabe wird gelöst durch einen Reaktor zur Durchführung einer Reaktion zwischen zwei nicht mischbaren Fluiden unterschiedlicher Dichte, mit

einem von einem zylindrischen, vertikal ausgerichteten länglichen Mantel, einem Boden und einer Haube gebildeten Innenraum,

wobei der Innenraum durch Einbauten in eine rückvermischte Zone, eine unterhalb der rückvermischten Zone angeordnete Zone begrenzter Rückvermischung und eine propfenförmig durchströmte Zone unterteilt ist, die zumindest von einem der Fluide nacheinander durchströmbar sind,

wobei die rückvermischte Zone wenigstens einen Einlass aufweist und die propfenförmig durchströmte Zone einen Auslass aufweist, und die rückvermischte Zone wenigstens ein Mischorgan aufweist, welches ausgewählt ist unter einem Rührer, einer Strahldüse und Mitteln zum Einpressen des Fluids geringerer Dichte,

einem ersten zylindrischen Einbauelement, welches sich im Innenraum in Längsrichtung des Reaktors erstreckt, welches die Zone begrenzter Rückvermischung von der propfenförmig durchströmten Zone abgrenzt und welches einen ersten Durchtritt zur rückvermischten Zone und einen zweiten Durchtritt zur propfenförmig durchströmten Zone aufweist, wobei das erste Einbauelement konzentrisch zum Mantel angeordnet ist, so dass die propfenförmig durchströmte Zone einen ringförmigen horizontalen Querschnitt aufweist, und wobei die untere Kante des ersten Einbauelements beabstandet zum Boden des Reaktors angeordnet ist,

einem zweiten Einbauelement, welches die rückvermischte Zone von der propfenförmig durchströmten Zone so abgrenzt, dass keine direkte Fluidverbindung zwischen der rückvermischten Zone und der propfenförmig durchströmten Zone besteht, wobei sich das zweite Einbauelement von der oberen Kante des ersten Einbauelements zum Mantel erstreckt, und wobei das zweite Einbauelement ein horizontal ausgerichtetes Ringblech ist, und

rückvermischungshindernden dritten Einbauelementen in Form von Füllkörpern, strukturierter Packungen oder flüssigkeitsdurchlässiger Böden, die in der Zone begrenzter Rückvermischung angeordnet sind.

Erfindungsgemäß ist die untere Kante des ersten Einbauelements beabstandet zum Boden des Reaktors angeordnet und bildet so den Durchtritt von der Zone begrenzter Rückvermischung zur propfenförmig durchströmten Zone.

Erfindungsgemäß erstreckt sich das zweite Einbauelement von der oberen Kante des ersten Einbauelements zum Mantel. Das zweite Einbauelement ist ein horizontal ausgerichtetes Ringblech. Das zweite Einbauelement teilt den Innenraum in vertikaler Richtung in eine obere Hälfte und eine untere Hälfte, z. B. im Verhältnis von oberer Hälfte zu unterer Hälfte von 4:1 bis 1:1, bevorzugt im Verhältnis von 3:1 bis 1:1, insbesondere im Verhältnis von 2:1 bis 1:1, beispielsweise 6:4.

Das Verhältnis von Länge zu Durchmesser des Reaktors beträgt üblicherweise 2:1 bis 100:1, bevorzugt 5:1 bis 50:1, besonders bevorzugt 7:1 bis 25:1.

Die Einspeisung der Fluide erfolgt an einer beliebigen Stelle der rückvermischten Zone. Die rückvermischte Zone weist wenigstens ein Mischorgan auf, welches ausgewählt ist unter einem Rührer, einer Strahldüse und Mitteln zum Einpressen des Fluids geringerer Dichte. Hierdurch werden die zwei nicht miteinander mischbare Fluide unterschiedlicher Dichte miteinander in intensiven Kontakt gebracht.

Fluid niedriger Dichte sammelt sich oberhalb des Trennspiegels der Fluide in der rückvermischten Zone. In einer bevorzugten Ausführungsform kann das nicht umgesetzte Fluid mit der geringeren Dichte über einen Fluidauslass aus der rückvermischten Zone entnommen werden.

In einer Ausführungsform ist das Mischorgan ein Rührer, z. B. ein Propeller-Rührer.

Alternativ kann die Einspeisung der Fluide so erfolgen, dass gleichzeitig eine Durchmischung der rückvermischten Zone bewirkt wird. Vorzugsweise erfolgt die Zufuhr des Fluids hoher Dichte (d. h. der Flüssigkeit bei gas/flüssig-Reaktionen) durch eine Strahldüse. Die Einspeisung durch eine Strahldüse kann oberhalb oder unterhalb des Trennspiegels der Fluide erfolgen, insbesondere durch eine oberhalb des Trennspiegels (Flüssigkeitsstandes) angeordnete, nach unten gerichtete Strahldüse.

Der Strahl aus der Düse sorgt für eine Dispergierung der Fluide niedriger Dichte im Reaktionsgemisch. Ist beispielsweise das erste Fluid eine Flüssigkeit und das zweite Fluid ein Gas, so fällt der Strahl aus der Düse durch den Leichtraum und schlägt beim Eintritt in die Flüssigphase das Gas ein, das auf diese Weise zu Bläschen zerteilt und in der Flüssigphase dispergiert wird.

Die Strahldüse kann als Einstoff- oder Zweistoffdüse ausgelegt werden. Bei der Einstoffdüse wird nur ein Fluid eingedüst. Vorteilhaft bei dieser Ausgestaltung ist der einfache Aufbau einer solchen Einstoffdüse. Bei der Zweistoffdüse werden miteinander nicht mischbare Fluide mit gegebenenfalls unterschiedlicher Dichte zusammen zugeführt und dispergiert.

Um das Fluid niedriger Dichte (Gas) zusammen mit dem Fluid hoher Dichte (Flüssigkeit) gemeinsam einzubringen, kann die Strahldüse als Mischdüse ausgebildet sein, z. B. eine Strahlsauger-Mischdüse (Flüssigkeitsstrahl-Ejektor). Der Begriff Mischdüse bezeichnet in üblicher Weise ein sich in Strömungsrichtung verjüngendes Rohr. Der austretende schnelle Strahl erzeugt in einem die Düse umgebenden Ansaugraum einen Unterdruck. Dadurch kann Fluid niedriger Dichte angesaugt und infolge des Impulsaustausches im Fluid hoher Dichte dispergiert und gemeinsam mit diesem in die rückvermischte Zone entlassen werden.

In einer weiteren Ausführungsform umfasst das Mischorgan Mittel zum Einpressen des Fluids geringerer Dichte in das Fluid höherer Dichte. Geeignete Mittel zum Einpressen des Fluids geringerer Dichte sind z. B. ein Kompressor zum Ansaugen und Komprimieren des Fluids niedriger Dichte (insbesondere Gas) oberhalb des Trennspiegels bzw. von Frischfluid und Düsen zum Einpressen des komprimierten Fluids unterhalb des Trennspiegels.

Der volumenspezifische Leistungseintrag in die rückvermischte Zone beträgt vorzugsweise 0,5 bis 5 $kW/m^3$. Der volumenspezifische Leistungseintrag kann bestimmt werden als Produkt aus Differenzdruck über die Strahldüse und Volumenstrom durch die Düse.

Vorzugsweise ist die rückvermischte Zone als Schlaufenreaktor ausgebildet. Beispiele für Schlaufenreaktoren sind Rohrreaktoren mit internen und externen Schlaufen. Solche Reaktoren sind beispielsweise in Ullmann's Encyclopädie näher beschrieben (Ullmann's Encyclopedia of Industrial Chemistry, Verlag Chemie, Electronic Release 2008, 7th Edition, Kapitel "Stirred Tank and Loop Reactors" und Kapitel "Bubble Columns").

Der Schlaufenreaktor ist im Allgemeinen als Rohrreaktor mit externem Kreislauf (externe Schlaufen) gestaltet. Bei einem Schlaufenreaktor mit externem Kreislauf befindet sich in der Regel ein Abzug an beliebiger Stelle der rückvermischten Zone, vorzugsweise im unteren Bereich der rückvermischten Zone, über den das Reaktionsgemisch in einem äußeren Kreislauf mittels eines Förderorgans der Strahldüse wieder zugeführt wird. Das Förderorgan ist vorzugsweise eine Pumpe, weshalb der externe Kreislauf üblicherweise als Umpumpkreislauf bezeichnet wird.

Beispiele für Pumpen sind Kreiselpumpen oder Rotationskolbenpumpen, wie Drehkolbenpumpen, Drehschieberpumpen, Kreiskolbenpumpen oder Zahnradpumpen. Besonders bevorzugt werden Kreiselpumpen als Förderorgan

verwendet.

Vorzugsweise ist die rückvermischte Zone als Schlaufenreaktor mit externem Kreislauf ausgebildet, wobei sich im externen Kreislauf ein Wärmeaustauscher befindet. Ein solcher Reaktor wird im Rahmen dieser Erfindung als Schlaufenreaktor mit externem Wärmetauscher bezeichnet.

Der Wärmetauscher ist beispielsweise ein Rohrbündelwärmetauscher, Doppelrohrwärmetauscher, Plattenwärmetauscher oder Spiralwärmetauscher. Bei Auslegungsdrücken für den Reaktor unter 100 bar wird bevorzugt ein Rohrbündelwärmetauscher verwendet, bei höheren Drücken wird bevorzugt ein oder mehrere hintereinander geschaltete Doppelrohrwärmetauscher verwendet.

Der Schlaufenreaktor mit externem Wärmetauscher wird dabei üblicherweise so betrieben, dass ein Teil des Reaktionsgemisches aus der rückvermischten Zone durch den externen Umpumpkreislauf, in dem sich der externe Wärmetauscher befindet, gefördert wird, wobei das durch den Wärmetauscher geförderte Reaktionsgemisch gekühlt wird. Durch das externe Umpumpen wird das Reaktionsgemisch in der ersten Reaktionsstufe in der Regel stark durchmischt und umgewälzt, so dass die Verweilzeit in der ersten Stufe üblicherweise der eines vollständig rückvermischten Rührkessels (CSTR) entspricht.

Das Reaktionsgemisch wird schließlich mittels der Strahldüse wieder der rückvermischten Zone zugeführt. Üblicherweise wird umzusetzendes Fluid und ggf. eine Katalysatorlösung in den Umpumpkreislauf dosiert und zusammen mit dem bereits im Umpumpkreislauf befindlichen Strom als Reaktionsgemisch der rückvermischten Zone des Reaktors zugeführt.

In einer bevorzugten Ausführungsform ist der Schlaufenreaktor so gestaltet, dass zusätzlich zum externen Kreislauf eine so genannte interne Schlaufenströmung ausgebildet wird. In einem Schlaufenreaktor mit interner Schlaufenströmung ist in der Regel im Inneren der rückvermischten Zone ein konzentrisches, vorzugsweise zylindrisches Leitrohr angeordnet, das sich im Wesentlichen über die gesamte Länge der rückvermischten Zone mit Ausnahmen des Bereichs der Haube und des Bereichs des zweiten Einbauelements erstreckt.

Das Leitrohr ist in der Regel als einfaches Rohr ausgestaltet. Das Verhältnis von Länge zu Durchmesser des Leitrohrs beträgt im Allgemeinen 5:1 bis 100:1, bevorzugt 5:1 bis 50:1.

Der Durchmesser des Leitrohrs ist geringer als der Durchmesser der rückvermischten Zone. Das Verhältnis des Durchmessers des Leitrohrs zum Durchmesser der rückvermischten Zone beträgt in der Regel 0,3:1 bis 0,9:1, bevorzugt 0,5:1 bis 0,7:1. Der Raum zwischen Leitrohr und dem Mantel wird im Allgemeinen als Ringraum bezeichnet.

Die Strahldüse ist üblicherweise so angeordnet, dass der von der Strahldüse erzeugte Fluidstrahl in das Leitrohr gerichtet ist. Die Strahldüse ist bevorzugt oberhalb des oberen Endes des Leitrohrs angeordnet. Die Düsenspitze der Strahldüse für das Fluid höherer Dichte befindet sich oberhalb des Flüssigkeitsstandes und taucht nicht in die Flüssigphase ein. Der mittels Strahldüse erzeugte Fluidstrahl bewirkt eine abwärts gerichtete Strömung im Leitrohr (Abstromsäule), die nach Verlassen des Leitrohrs umgelenkt wird, so dass die Flüssigkeit im Ringraum zwischen Leitrohr und Mantel in Richtung der Strahldüse wieder aufströmt (Aufstromsäule). So entsteht in der Regel eine interne Schlaufenströmung. Das Verhältnis der Volumenströme von interner Schlaufenströmung zu extern umgepumpten Reaktionsgemisch beträgt vorzugsweise 2 bis 30:1, besonders bevorzugt 5 bis 20:1.

Aus der rückvermischten Zone wird zumindest ein Teil des Reaktionsgemisches der Zone begrenzter Rückvermischung zugeführt. In der Zone begrenzter Rückvermischung ist die Verweilzeitverteilung der Verweilzeitverteilung eines Rohrreaktors angenähert. Durch diese definierte Flüssigkeitsverweilzeit kann der Umsatz des Edukts gesteigert werden.

Das Einstellen des Volumenverhältnisses von rückvermischter Zone zur Zone begrenzter Rückvermischung ermöglicht eine Anpassung der Verweilzeitverteilung je nach Anforderungen der durchzuführenden Reaktion. Das Volumenverhältnis von rückvermischter Zone zur Zone begrenzter Rückvermischung liegt vorzugswiese im Bereich von 9:1 bis 6:4, insbesondere im Bereich von 8:2 bis 6:4 und ganz besonders bevorzugt im Bereich von 7:3 bis 6:4, beispielsweise 7:3.

Die Rückvermischung im Reaktor wird durch in der Zone begrenzter Rückvermischung angeordnete rückvermischungshindernde dritte Einbauelemente begrenzt. Durch den Einbau solcher Elemente werden in der Regel die Zirkulation und damit die Rückvermischung von Fluiden unterschiedlicher Dichte, beispielsweise Gas und Flüssigkeit, eingeschränkt.

Die Begrenzung der Rückvermischung im Reaktor wird durch den Einbau von Füllkörpern, strukturierten Packungen oder flüssigkeitsdurchlässigen Böden realisiert. In einer bevorzugten Ausführungsform erfolgt die Begrenzung der Rückvermischung durch den Einbau von mehreren, fest angeordneten, flüssigkeitsdurchlässigen Böden in das erste zylindrische Einbauelement. Es entstehen zwischen den einzelnen Böden somit Einzelsegmente ("Kompartimente") mit definierten Reaktionsvolumina. Jedes der Einzelsegmente wirkt in der Regel dabei wie ein einzelner, rückvermischter Rührkesselreaktor. Mit zunehmender Anzahl von Einzelsegmenten hintereinander nähert sich die Verweilzeitverteilung einer derartigen Kaskade in der Regel der Verweilzeit eines Rohrreaktors an.

Die Anzahl der so gebildeten Einzelsegmente beträgt vorzugsweise 2 bis 20, besonders bevorzugt 2 bis 10, insbesondere bevorzugt 3 bis 6. Besonders bevorzugt sind die Böden als Lochbleche gestaltet.

In einer weiteren Ausführungsform erfolgt die Begrenzung der Rückvermischung durch den Einbau von Füllkörpern. Die Füllkörper können unterschiedliche Formen aufweisen und sind meist etwa 2 bis 15 mm groß. Bekannte Beispiele sind kugelförmige und zylinderförmige Vollkörper, Raschig-Ringe (Hohlzylinder), Pall-Ringe, Hiflow-Ringe, Intalox-Sattel und dergleichen. Vorzugsweise sind die Füllkörper Vollkörper. Die Füllkörper können geordnet, aber auch regellos (als Schüttung) in die Zone begrenzter Rückvermischung eingebracht werden. Als Material können Glas, Keramik, Metall und Kunststoffe verwendet werden.

In einer weiteren Ausführungsform erfolgt die Begrenzung der Rückvermischung durch den Einbau von strukturierten Packungen. Strukturierte Packungen sind eine Weiterentwicklung der geordneten Füllkörper. Sie weisen eine regelmäßig geformte Struktur auf. Es gibt verschiedene Ausführungen von Packungen, wie Gewebe- oder Blechpackungen. Als Material können Metall, Kunststoff, Glas und Keramik verwendet werden.

Im Falle einer gas/flüssig-zweiphasigen Reaktion erfolgt die Reaktion in einem zum Austritt aus der Zone begrenzter Rückvermischung gelegenen Abschnitt der Zone begrenzter Rückvermischung vorteilhafterweise flüssig-einphasig, d. h. es liegt im zum Austritt aus der Zone begrenzter Rückvermischung gelegenen Abschnitt keine dispergierte Gasphase vor und die Reaktion erfolgt ausschließlich mit dem in der Flüssigphase gelösten Gas. Beispielsweise kann in dem Reaktor eine Hydrierung mit Wasserstoffgas durchgeführt werden.

Da die Hydrierumsätze zum Austritt aus der Zone begrenzter Rückvermischung hin üblicherweise gering sind, ist die Konzentration an gelöstem Wasserstoff ausreichend. Durch die Abwesenheit einer diskreten Gasphase zum Austritt aus der Zone begrenzter Rückvermischung hin kann der Flüssigkeits-Holdup der Zone begrenzter Rückvermischung vergrößert und die Verweilzeit der Flüssigphase in der Zone begrenzter Rückvermischung erhöht werden. Da die Hydrierung in der Flüssigphase erfolgt, wird der Reaktionsraum auf diese Weise optimal genutzt.

Auf den flüssig-einphasig betriebenen Abschnitt der Zone begrenzter Rückvermischung entfallen vorzugsweise 30 bis 50% des Gesamtvolumens der Zone begrenzter Rückvermischung.

Aus der Zone begrenzter Rückvermischung gelangt das Reaktionsgemisch schließlich in die propfenförmig durchströmten Zone. In der propfenförmig durchströmten Zone ist die Rückvermischung begrenzt, so dass die Verweilzeitverteilung in dieser Zone der eines Rohrreaktors weiter angenähert ist. Durch diese definierte Flüssigkeitsverweilzeit kann der Umsatz des Edukts nahezu vervollständigt werden.

Zumindest am Austritt aus der Zone begrenzter Rückvermischung, also beim Eintritt in die propfenförmig durchströmte Zone, liegt im Falle einer gas/flüssig-zweiphasigen Reaktion vorzugsweise keine dispergierte Gasphase vor und die Reaktion erfolgt ausschließlich mit dem in der Flüssigphase gelösten Gas. Da die Umsätze in der propfenförmig durchströmten Zone üblicherweise gering sind, ist die Konzentration an gelöstem Gas ausreichend. Durch die Abwesenheit einer diskreten Gasphase in der propfenförmig durchströmten Zone kann der Flüssigkeits-Holdup vergrößert und die Verweilzeit der Flüssigphase in der propfenförmig durchströmten Zone erhöht werden. Der Reaktionsraum wird auf diese Weise optimal genutzt.

Das Volumenverhältnis von der Zone begrenzter Rückvermischung zur propfenförmig durchströmten Zone liegt vorzugswiese im Bereich von 7:1 bis 3:1, besonders bevorzugt im Bereich von 5:1 bis 3:1 und ganz besonders bevorzugt im Bereich von 4:1 bis 3:1, beispielsweise 3:1.

Vorzugsweise ist die propfenförmig durchströmte Zone so ausgestaltet, dass sie turbulent durchströmt wird. Unter einer turbulenten Strömung wird hier ein Wert der Reynolds-Zahl Re von mehr als 3000 verstanden. Es gilt also:

$$Re = \frac{\rho v d}{\eta} > 2000$$

wobei $\rho$ für die Dichte des Reaktionsgemisches steht, v für die Strömungsgeschwindigkeit des Reaktionsgemisches steht, d für die charakteristische Länge der propfenförmig durchströmten Zone steht und $\eta$ für die dynamische Viskosität des Reaktionsgemischs steht. Die charakteristische Länge der propfenförmig durchströmten Zone ist der hydraulische Durchmesser, nämlich 2 x die Spaltbreite.

Aus der vorstehenden Diskussion ergibt sich, dass die Turbulenz umso höher ist, je geringer die Ringdicke ist. Die Ringdicke beträgt aus konstruktiven Gründen vorzugsweise mindestens 2 cm.

Der Druckunterschied zwischen der rückvermischten Zone, der Zone begrenzter Rückvermischung und der propfenförmig durchströmten Zone entspricht nur dem Druckverlust, der beim Durchströmen der rückvermischungshindernden dritten Einbauelemente der Zone begrenzter Rückvermischung entsteht. Vorteilhafterweise können die ersten, zweiten und dritten Einbauelemente daher unabhängig vom für das durchzuführende Verfahren vorgesehenen Druck ausgestaltet werden.

Die Erfindung betrifft außerdem ein Verfahren zur Durchführung einer kontinuierlichen Reaktion, bei dem man

- ein erstes Fluid höherer Dichte und ein zweites Fluid geringerer Dichte in die rückvermischte Zone eines Reaktors nach einem der vorhergehenden Ansprüche einführt, so dass zumindest das erste Fluid die rückvermischte Zone,

die Zone begrenzter Rückvermischung und die propfenförmig durchströmte Zone nacheinander durchströmt, und

- das ein Reaktionsprodukt enthaltende erste Fluid am Reaktionsprodukt-Auslass der propfenförmig durchströmten Zone abzieht.

Vorzugsweise wird das Verfahren so ausgeführt, dass man nicht umgesetztes zweites Fluid über einen Auslass aus der rückvermischten Zone zumindest teilweise ausführt. Besonders bevorzugt ist das erste Fluid eine Flüssigkeit und das zweite Fluid ein Gas.

Vorzugsweise dispergiert man das Gas in der rückvermischten Zone in der Flüssigkeit, wobei die Flüssigkeit die Zone begrenzter Rückvermischung und die propfenförmig durchströmte Zone nacheinander durchströmt, wobei das Volumenverhältnis von dispergiertem Gas zur Flüssigkeit in Strömungsrichtung von der Zone begrenzter Rückvermischung zur propfenförmig durchströmten Zone abnimmt, so dass die propfenförmig durchströmte Zone von der im Wesentlichen einphasigen Flüssigkeit durchströmt wird.

In einer Ausführungsform handelt es sich bei dem Verfahren um ein Verfahren zur Durchführung einer Hochdruckreaktion. Unter einer Hochdruckreaktion wird eine Reaktion verstanden, die bei einem gegenüber Umgebungsdruck erhöhtem Druck durchgeführt wird, beispielsweise bei mindestens 5 bar absolut, mindestens 20 bar absolut oder mindestens 50 bar absolut.

Vorzugsweise nimmt das Volumenverhältnis von im Reaktionsgemisch dispergierten Gas zur Flüssigkeit in Durchströmungsrichtung der Zone begrenzter Rückvermischung ab, so dass eine im Wesentlichen einphasige Flüssigkeit aus der Zone begrenzter Rückvermischung austritt.

In einer bevorzugten Ausführungsform ist das Gas Wasserstoff. Besonders bevorzugt ist ein Verfahren zur Herstellung einer optisch aktiven Carbonylverbindung durch asymmetrische Hydrierung einer prochiralen, α,β-ungesättigten Carbonylverbindung mit Wasserstoff in Gegenwart eines homogenen Rhodium-Katalysators, der wenigstens einen chiralen Liganden aufweist.

Eine prochirale α,β-ungesättigte Carbonylverbindung kann durch Additionsreaktion an die olefinische Doppelbindung ein Chiralitätszentrum ausbilden. Hierzu trägt die Doppelbindung vier verschiedene Substituenten. Die prochirale α,β-ungesättigte Carbonylverbindung ist vorzugsweise ausgewählt unter Verbindungen der allgemeinen Formel (I)

(I) 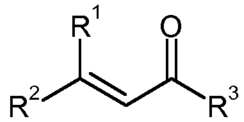

worin

$R^1$, $R^2$ voneinander verschieden sind und jeweils für einen unverzweigten, verzweigten oder cyclischen Kohlenwasserstoffrest mit 1 bis 25 Kohlenstoffatomen stehen, der gesättigt ist oder eine oder mehrere, nicht konjugierte ethylenische Doppelbindungen aufweist, und der unsubstituiert ist oder einen oder mehrere gleiche oder verschiedene Substituenten trägt, die ausgewählt sind unter $OR^4$, $NR^{5a}R^{5b}$, Halogen, $C_6$-$C_{10}$-Aryl und Hetaryl mit 5 bis 10 Ringatomen,

$R^3$ für Wasserstoff oder für einen unverzweigten, verzweigten oder cyclischen Kohlenwasserstoffrest mit 1 bis 25 Kohlenstoffatomen steht, der gesättigt ist oder eine oder mehrere, nicht konjugierte ethylenische Doppelbindungen aufweist, und der unsubstituiert ist oder einen oder mehrere gleiche oder verschiedene Substituenten trägt, die ausgewählt sind unter $OR^4$, $NR^{5a}R^{5b}$, Halogen, $C_6$-$C_{10}$-Aryl und Hetaryl mit 5 bis 10 Ringatomen,

oder

$R^3$ gemeinsam mit einem der Reste $R^1$ oder $R^2$ auch eine 3 bis 25-gliedrige Alkylengruppe bedeuten kann worin 1, 2, 3 oder 4 nicht benachbarte $CH_2$-Gruppen durch O oder N-$R^{5c}$ ersetzt sein können, wobei die Alkylengruppe gesättigt ist oder eine oder mehrere, nicht konjugierte ethylenische Doppelbindungen aufweist, und wobei die Alkylengruppe unsubstituiert ist oder einen oder mehrere gleiche oder verschiedene Substituenten trägt, die ausgewählt sind unter $OR^4$, $NR^{5a}R^{5b}$, Halogen, $C_1$-$C_4$-Alkyl, $C_6$-$C_{10}$-Aryl und Hetaryl mit 5 bis 10 Ringatomen, wobei zwei Substituenten auch gemeinsam für eine 2 bis 10-gliedrige Alkylengruppe stehen können, wobei die 2- bis 10-gliedrige Alkylengruppe gesättigt ist oder eine oder mehrere, nicht konjugierte ethylenische Doppelbindungen

aufweist, und wobei die 2- bis 10-gliedrige Alkylengruppe unsubstituiert ist oder einen oder mehrere gleiche oder verschiedene Substituenten trägt, die ausgewählt sind unter $OR^4$, $NR^{5a}R^{5b}$, Halogen, $C_6$-$C_{10}$-Aryl und Hetaryl mit 5 bis 10 Ringatomen;

wobei

$R^4$ für Wasserstoff, $C_1$-$C_6$-Alkyl, $C_6$-$C_{10}$-Aryl, $C_6$-$C_{14}$-Aryl-$C_1$-$C_{10}$-alkyl, oder $C_1$-$C_{10}$-Alkyl-$C_6$-$C_{14}$-arylsteht;

$R^{5a}$, $R^{5b}$ jeweils unabhängig voneinander Wasserstoff, $C_1$-$C_6$-Alkyl, $C_6$-$C_{10}$-Aryl, $C_6$-$C_{14}$-Aryl-$C_1$-$C_{10}$-alkyl oder $C_1$-$C_{10}$-Alkyl-$C_6$-$C_{14}$-aryl bedeuten oder

$R^{5a}$ und $R^{5b}$ gemeinsam auch eine Alkylenkette mit 2 bis 5 Kohlenstoffatomen, die durch N oder O unterbrochen sein kann, bedeuten können; und

$R^{5c}$ für Wasserstoff, $C_1$-$C_6$-Alkyl, $C_6$-$C_{10}$-Aryl, $C_6$-$C_{14}$-Aryl-$C_1$-$C_{10}$-alkyl oder $C_1$-$C_{10}$-Alkyl-$C_6$-$C_{14}$-ary- steht.

In bevorzugten Ausführungsformen ist die prochirale α,β-ungesättigte Carbonylverbindung ausgewählt unter Verbindungen der allgemeinen Formeln (Ia) und (Ib)

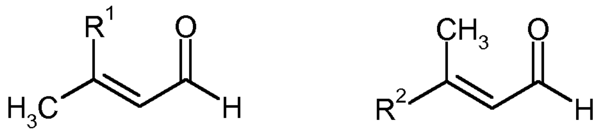

(Ia) (Ib)

worin

$R^1$, $R^2$ jeweils für einen unverzweigten oder verzweigten Kohlenwasserstoffrest mit 2 bis 25 Kohlenstoffatomen steht, der gesättigt ist oder 1, 2, 3, 4 oder 5 nicht konjugierte ethylenische Doppelbindungen aufweist.

Eine besonders bevorzugte Ausführungsform betrifft ein Verfahren zur Herstellung von optisch aktivem Citronellal der Formel (III)

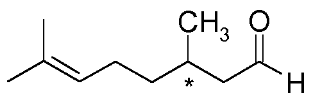

(III)

worin * das Asymmetriezentrum bezeichnet;
durch asymmetrische Hydrierung von Geranial der Formel (Ia-1) oder von Neral der Formel (Ib-1)

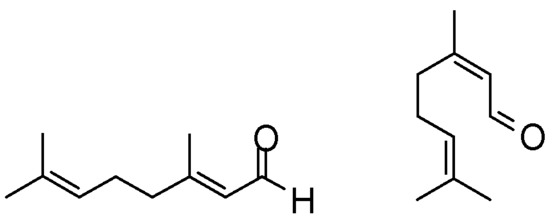

(Ia-1) (Ib-1)

oder einer Neral und Geranial enthaltenden Mischung. Eine Neral und Geranial enthaltende Mischung ist unter der Bezeichnung Citral bekannt.

Man kann das so erhältliche optisch aktive Citronellal der Formel (III) einer Cyclisierung zu optisch aktivem Isopulegol unterziehen und das optisch aktive Isopulegol zu optisch aktivem Menthol hydrieren.

Durch dieses Verfahren zur Herstellung einer optisch aktiven Carbonylverbindung gelingt es, optisch aktive

Carbonylverbindungen, insbesondere optisch aktive Aldehyde in hohen Ausbeuten und Enantiomerenüberschüssen bereitzustellen. Üblicherweise erhält man die gewünschten asymmetrisch hydrierten Verbindungen in einem Enantiomerenüberschuss von mindestens 80 % ee, oft mit einem Enantiomerenüberschuss von etwa 85 bis etwa 99 % ee. Dabei ist zu beachten, dass der maximal erzielbare Enantiomerenüberschuss von der Reinheit des eingesetzten Substrats, insbesondere im Hinblick auf die Isomerenreinheit der zu hydrierenden Doppelbindung, abhängen kann. Demzufolge eignen sich als Ausgangssubstanzen insbesondere solche, die ein Isomerenverhältnis von mindestens etwa 90:10, bevorzugt mindestens etwa 95:5 bezüglich der E/Z-Doppelbindungsisomere aufweisen.

Dieses bevorzugte Verfahren zur Herstellung einer optisch aktiven Carbonylverbindung wird in Gegenwart eines im Reaktionsgemisch löslichen, optisch aktiven Rhodium-Katalysators durchgeführt, der mindestens einen optisch aktiven Liganden aufweist. Derartige Katalysatoren sind beispielsweise erhältlich durch Reaktion einer geeigneten, im Reaktionsgemisch löslichen Rhodium-Verbindung mit einem optisch aktiven Liganden, der mindestens ein Phosphor- und/oder Arsenatom aufweist.

Beispiele für erfindungsgemäß einsetzbare Rhodiumverbindungen sind: $RhCl_3$, $Rh(OAc)_3$, $[Rh(cod)Cl]_2$, $Rh(CO)_2acac$, $[Rh(cod)OH]_2$, $[Rh(cod)OMe]_2$, $Rh_4(CO)_{12}$, $Rh_6(CO)_{16}$, wobei "acac" für einen Acetylacetonat- und "cod" für einen Cyclooctadien-Liganden steht.

Die Katalysatorkonzentration im Reaktionsgemisch beträgt vorzugsweise 0,001 bis 1 mol-%, insbesondere 0,002 bis 0,5 mol-%, besonders bevorzugt 0,005 bis 0,2 mol-%, bezogen auf die Menge an prochiraler $\alpha,\beta$-ungesättigter Carbonylverbindung im Reaktionsgemisch, gerechnet als im Katalysator enthaltene Rhodium-Atome.

Die genannten Rhodiumverbindungen werden mit einer weiteren Verbindung in Kontakt gebracht, die optisch aktiv, bevorzugt im wesentlichen enantiomerenrein ist (d.h. einen Enantiomerenüberschuss von mindestens etwa 99% aufweist) und mindestens ein Phosphor und/oder Arsenatom, bevorzugt mindestens ein Phosphoratom aufweist. Diese als chiraler Ligand zu bezeichnende Verbindung bildet im Reaktionsgemisch bzw. im Präformierungsgemisch mit der eingesetzten Rhodium-Verbindung den erfindungsgemäß einzusetzenden Rhodium-Katalysator.

Insbesondere bevorzugt sind solche chiralen Liganden die zwei Phosphoratome aufweisen und mit Rhodium Chelatkomplexe bilden.

Als chirale Liganden eignen sich im Rahmen der vorliegenden Erfindung solche Verbindungen, wie sie beispielsweise in: I. Ojima (Hrsg.), Catalytic Asymmetric Synthesis, Wiley-VCh, 2. Auflage, 2000 oder in E. N. Jacobsen, A. Pfaltz, H. Yamamoto (Hrsg.), Comprehensive Asymmetric Catalysis, 2000, Springer oder in W. Tang, X. Zhang, Chem. Rev. 2003, 103, 3029-3069 beschrieben sind.

Bevorzugte Liganden sind chirale zweizähnige Bisphosphin-Liganden, insbesondere solche der allgemeinen Formeln (IV) bis (VI)

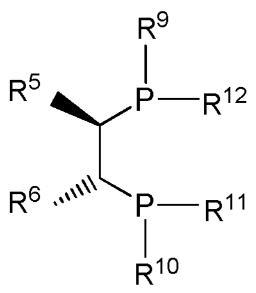
(IV)

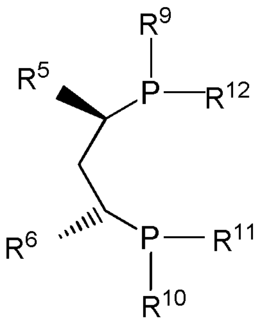
(V)

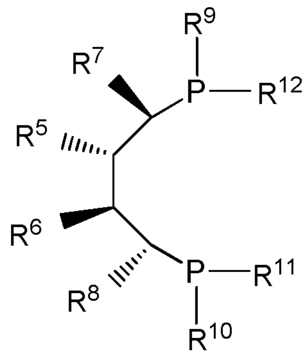
(VI)

worin

$R^5$, $R^6$ jeweils unabhängig voneinander für einen unverzweigten, verzweigten oder cyclischen Kohlenwasserstoffrest mit 1 bis 20 Kohlenstoffatomen stehen, der gesättigt ist oder eine oder mehrere, in der Regel 1 bis etwa 4, nicht konjugierte, ethylenische Doppelbindungen aufweisen kann und der unsubstituiert ist oder einen oder mehrere, in der Regel 1 bis 4, gleiche oder verschiedene Substituenten trägt, die ausgewählt sind unter $OR^{13}$, $NR^{14}R^{15}$, Halogen, $C_6$-$C_{10}$-Aryl und $C_3$-$C_9$-Hetaryl, oder

$R^5$ und $R^6$ gemeinsam eine 2 bis 10-gliedrige Alkylengruppe oder eine 3 bis 10-gliedrige Cycloalkylengruppe bedeuten kann, worin 1 , 2, 3 oder 4 nicht benachbarte CH-Gruppen durch O oder N-$R^{13}$ ersetzt sein können, wobei die Alkylengruppe und die Cycloalkylengruppe gesättigt sind oder eine oder zwei, nicht konjugierte ethylenische

Doppelbindungen aufweisen, und wobei die Alkylengruppe und die Cycloalkylengruppe unsubstituiert sind oder einen oder mehrere gleiche oder verschiedene Substituenten tragen, die ausgewählt sind unter $C_1$-$C_4$-Alkyl;

$R^7$, $R^8$ jeweils unabhängig voneinander Wasserstoff oder geradkettiges oder verzweigtes $C_1$-$C_4$-Alkyl bedeuten und

$R^9$, $R^{10}$, $R^{11}$, $R^{12}$ gleich oder verschieden sind und für $C_6$-$C_{10}$-Aryl stehen, das unsubstituiert ist oder einen oder mehrere Substituenten trägt, die ausgewählt sind unter $C_1$-$C_6$-Alkyl, $C_3$-$C_6$-Cycloalkyl, $C_6$-$C_{10}$-Aryl, $C_1$-$C_6$-Alkoxy und Amino;

$R^{13}$, $R^{14}$, $R^{15}$ jeweils unabhängig voneinander Wasserstoff, $C_1$-$C_4$-Alkyl, $C_6$-$C_{10}$-Aryl, $C_7$-$C_{12}$-Aralkyl oder $C_7$-$C_{12}$-Alkylaryl bedeuten, wobei $R^{14}$ und $R^{15}$ auch gemeinsam eine Alkylenkette mit 2 bis 5 Kohlenstoffatomen, die durch N oder O unterbrochen sein kann, bedeuten können.

Im Hinblick auf die Formeln (IV), (V) und (VI) haben die Variablen insbesondere die folgende Bedeutung:

$R^5$, $R^6$ stehen jeweils unabhängig voneinander für $C_1$-$C_4$-Alkyl oder

$R^5$ und $R^6$ stehen gemeinsam für einen $C_3$-$C_5$-Alkandiyl-Rest, $C_3$-$C_7$-Alkendiyl-Rest, $C_5$-$C_7$-Cycloalkandiyl-Rest oder einen $C_5$-$C_7$-Cycloalkendiyl-Rest, wobei die vier vorgenannten Reste unsubstituiert sind oder einen oder mehrere gleiche oder verschiedene Substituenten tragen, die ausgewählt sind unter $C_1$-$C_4$-Alkyl;

$R^7$, $R^8$ stehen jeweils unabhängig voneinander Wasserstoff oder $C_1$-$C_4$-Alkyl;

$R^9$, $R^{10}$, $R^{11}$, $R^{12}$ stehen jeweils für Phenyl.

Aufgrund ihrer leichten Verfügbarkeit besonders bevorzugte chirale, zweizähnige Bisphosphin-Liganden sind unter der Bezeichnung "Chiraphos" erhältliche Verbindungen der Formel:

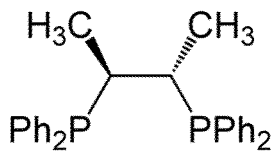

Die chiralen Liganden werden vorteilhaft in einer Menge von etwa 0,9 bis etwa 10 mol, bevorzugt etwa 1 bis etwa 4 mol pro mol eingesetzter Rhodium-Verbindung eingesetzt. Geeigneterweise wird der im Reaktionsgemisch lösliche, optisch aktive Rhodium-Katalysator durch Umsetzung einer achiralen Rhodiumverbindung und mit einem chiralen, zweizähnigen Bisphosphin-Liganden vor oder während der Hydrierung in-situ generiert. In diesem Zusammenhang bedeutet der Ausdruck "in-situ", dass der Katalysator direkt vor oder zu Beginn der Hydrierung generiert wird. Bevorzugt wird der Katalysator vor der Hydrierung generiert.

Es wurde gefunden, dass die Gegenwart einzähniger Liganden die Aktivität des Katalysators erhöhen kann. In einer bevorzugten Ausführungsform des erfindungsgemäßen Verfahrens werden Verbindungen der Formel (II)

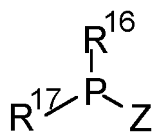
(II)

eingesetzt, worin Z in Formel (II) für eine Gruppe $CHR^{18}R^{19}$ steht und worin die Variablen $R^{16}$, $R^{17}$, $R^{18}$, $R^{19}$ unabhängig voneinander und insbesondere gemeinsam die folgenden Bedeutungen aufweisen:

$R^{16}$, $R^{17}$: sind gleich oder verschieden und stehen für Phenyl, das unsubstituiert ist oder 1, 2 oder 3 Substituenten trägt, die ausgewählt sind unter Methyl und Methoxy, wobei $R^{16}$ und $R^{17}$ jeweils insbesondere für unsubstituiertes Phenyl stehen;

$R^{18}$ steht für $C_1$- bis $C_4$-Alkyl, insbesondere für Methyl;

$R^{19}$ steht für $C_1$- bis $C_4$-Alkyl, das eine Gruppe $P(=O)R^{19a}R^{19b}$ trägt, wobei und insbesondere eine Gruppe $CH_2$-$P(=O)R^{19a}R^{19b}$ oder $CH(CH_3)$-$P(=O)R^{19a}R^{19b}$ bedeutet;

wobei

$R^{19a}$, $R^{19b}$: gleich oder verschieden sind und für Phenyl stehen, das unsubstituiert ist oder 1, 2 oder 3 Substituenten trägt, die ausgewählt sind unter Methyl und Methoxy, wobei besonders bevorzugt $R^{19a}$ und $R^{19b}$ jeweils für unsubstituiertes Phenyl stehen.

In dieser bevorzugten Ausführungsform des erfindungsgemäßen Verfahrens wird besonders bevorzugt eine Verbindung der Formel (II) eingesetzt, worin

$R^{16}$, $R^{17}$: für unsubstituiertes Phenyl stehen;

$R^{18}$ für Methyl steht;

$R^{19}$ eine Gruppe $CH(CH_3)$-$P(=O)R^{19a}R^{19b}$ bedeutet, worin $R^{19a}$ und $R^{19b}$ jeweils für unsubstituiertes Phenyl stehen.

Hierbei handelt es sich um die Verbindung (2-(Diphenylphosphoryl)-1-methylpropyl)-diphenylphosphan (Chiraphosmonooxid), einschließlich des (R,R)-Enantiomers (= (R,R)-Chiraphosmonooxid) und des (S,S)-Enantiomers (= (S,S)-Chiraphosmonooxid) sowie Gemischen aus (R,R)-Chiraphosmonooxid und (S,S)-Chiraphosmonooxid.

Falls die Reste $R^{18}$ und $R^{19}$ in der allgemeinen Formel (II) unterschiedliche Bedeutung haben, kann das Kohlenstoffatom, welches die Reste $R^{18}$ und $R^{19}$ trägt, eine (R)- oder (S)-Konfiguration aufweisen. Diese Verbindungen der allgemeinen Formel (II) können als reine (R)- oder reine (S)-Stereoisomere oder als Mischungen davon vorliegen. In der Regel wird man in diesen Fällen die reinen (R)- und (S)-Stereoisomere einsetzen, wobei auch etwaige Stereoisomerengemische für den Einsatz in dem vorliegenden Verfahren geeignet sind.

Unter einem reinen Stereoisomeren versteht man hier und im Folgenden chirale Substanzen, die bezüglich des gewünschten Stereoisomeren einen Enantiomerenüberschuss (ee) von wenigstens 80%ee, insbesondere wenigstens 90%ee und speziell wenigstens 95%ee aufweisen.

Insbesondere wird als chiraler Ligand Chiraphos verwendet und als einzähnig bindende Verbindung (2-(Diphenylphosphoryl)-1 -methylpropyl)-diphenylphosphan (Chiraphosmonooxid). Beispielsweise wird als chiraler Ligand R-Chiraphos verwendet und als einzähnig bindende Verbindung (R,R)-Chiraphosmonooxid und/oder (S,S)-Chiraphosmonooxid. Alternativ wird als chiraler Ligand S-Chiraphos verwendet und als einzähnig bindende Verbindung (R,R)-Chiraphosmonooxid und/oder (S,S)-Chiraphosmonooxid.

Die Verbindung der Formel (II) wird erfindungsgemäß in der Regel in einer Menge von 0,01 bis 1 Mol, bevorzugt 0,02 bis 0,8 Mol, besonders bevorzugt 0,03 bis 0,7 Mol und insbesondere in einer Menge von 0,04 bis 0,6 Mol pro Mol Rhodium einsetzt.

Weitere Ausführungsformen des Hydrierkatalysators und des einzähnigen Liganden sind in US 2018/0057437 A1, WO 2006/040096 A1 und WO 2008/132057 A1 beschrieben.

In einer Ausführungsform werden zwei miteinander nicht mischbare Flüssigkeiten unterschiedlicher Dichte in den Reaktor eingeführt, die ein zweiphasiges Reaktionsgemisch bilden. Beispielsweise ist das erste Fluid eine nicht wasser-mischbare organische Flüssigkeit und das zweite Fluid eine wässrige Flüssigkeit.

In einer Ausführungsform handelt es sich bei dem Verfahren um ein Verfahren zur Herstellung eines β-Hydroxyketons, wobei erste Fluid ein Dialkylketon umfasst und das zweite Fluid eine Formaldehyd-Quelle umfasst.

Das Dialkylketon ist vorzugsweise ausgewählt unter Dimethlketon, Diethylketon und Dipropylketon und ist insbesondere Diethylketon. Die Formaldehyd-Quelle stellt Formaldehyd in flüssiger Phase oder als Gasphase bereit. Vorzugsweise ist die Formaldehyd-Quelle eine wässrige Formaldehyd-Lösung. Beispielsweise umfasst die wässrige Formaldehyd-Lösung mindestens 15 Gew.-%, insbesondere mindestens 25 Gew.-% oder mindestens 35 Gew.-% oder mindestens 45 Gew.-% Formaldehyd.

Bevorzugt wird auf diese Weise 1-Hydroxy-2-methyl-3-pentanon (HMP) hergestellt, wobei das erste Fluid Diethylketon umfasst und das zweite Fluid eine wässrige Formaldehyd-Lösung umfasst.

Ferner kommen Hydrolysereaktionen in Betracht, wobei das erste Fluid eine mit Wasser nicht-mischbare Flüssigkeit umfasst und das zweite Fluid wässrige Natronlauge umfasst.

Die Erfindung wird durch die beigefügte Figur näher veranschaulicht.

Fig. 1 zeigt schematisch eine erfindungsgemäßen Reaktor zur Durchführung eines erfindungsgemäßen Verfahrens.

Gemäß Fig. 1 ist ein erfindungsgemäßer Reaktor durch Einbauten in eine rückvermischte Zone 101, eine Zone

begrenzter Rückvermischung 102 und eine pfropfenförmig durchströmte Zone 103 unterteilt. Der Reaktor umfasst einen Rührer 104. Über die Leitung 105 wird dem Reaktor ein erstes Fluid höherer Dichte zugeführt, beispielsweise eine Flüssigkeit. Über die Leitung 106 wird dem Reaktor ein zweites Fluid geringerer Dichte zugeführt, beispielsweise ein Gas. Der Reaktor ist so ausgestaltet, dass das Reaktionsgmisch die rückvermischte Zone 101, die Zone begrenzter Rückvermischung 102 und die pfropfenförmig durchströmte Zone 103 nacheinander durchströmt. Bei der Reaktion gegebenenfalls anfallendes Abgas wird an einem Fluidauslass über Leitung 107 aus dem Reaktor ausgeführt. Das Reaktionsprodukt wird an einem Reaktionsproduktaulass über Leitung 108 abgezogen.

**Patentansprüche**

**1.** Reaktor zur Durchführung einer Reaktion zwischen zwei nicht mischbaren Fluiden unterschiedlicher Dichte, mit

einem von einem zylindrischen, vertikal ausgerichteten länglichen Mantel, einem Boden und einer Haube gebildeten Innenraum,
wobei der Innenraum durch Einbauten in eine rückvermischte Zone (101), eine unterhalb der rückvermischten Zone (101) angeordnete Zone begrenzter Rückvermischung (102) und eine propfenförmig durchströmte Zone (103) unterteilt ist, die zumindest von einem der Fluide nacheinander durchströmbar sind,
wobei die rückvermischte Zone (101) wenigstens einen Einlass aufweist und die propfenförmig durchströmte Zone (103) einen Auslass aufweist, und die rückvermischte Zone (101) wenigstens ein Mischorgan (104) aufweist, welches ausgewählt ist unter einem Rührer, einer Strahldüse und Mitteln zum Einpressen des Fluids geringerer Dichte,
einem ersten zylindrischen Einbauelement, welches sich im Innenraum in Längsrichtung des Reaktors erstreckt, welches die Zone begrenzter Rückvermischung (102) von der propfenförmig durchströmten Zone (103) abgrenzt und welches einen ersten Durchtritt zur rückvermischten Zone (101) und einen zweiten Durchtritt zur propfenförmig durchströmten Zone (103) aufweist, wobei das erste Einbauelement konzentrisch zum Mantel angeordnet ist, so dass die propfenförmig durchströmte Zone (103) einen ringförmigen horizontalen Querschnitt aufweist, und wobei die untere Kante des ersten Einbauelements beabstandet zum Boden des Reaktors angeordnet ist,
einem zweiten Einbauelement, welches die rückvermischte Zone (101) von der propfenförmig durchströmten Zone (103) so abgrenzt, dass keine direkte Fluidverbindung zwischen der rückvermischten Zone (101) und der propfenförmig durchströmten Zone (103) besteht, wobei sich das zweite Einbauelement von der oberen Kante des ersten Einbauelements zum Mantel erstreckt, und wobei das zweite Einbauelement ein horizontal ausgerichtetes Ringblech ist, und
rückvermischungshindernden dritten Einbauelementen in Form von Füllkörpern, strukturierten Packungen oder flüssigkeitsdurchlässigen Böden, die in der Zone begrenzter Rückvermischung (102) angeordnet sind.

**2.** Reaktor nach Anspruch 1, wobei die propfenförmig durchströmte Zone (103) so angeordnet ist, dass sie turbulent durchströmt wird.

**3.** Reaktor nach einem der vorhergehenden Ansprüche, wobei das zweite Einbauelement den Innenraum in vertikaler Richtung in eine obere Hälfte und eine untere Hälfte im Verhältnis von 4:1 bis 1:1 teilt.

**4.** Reaktor nach einem der vorhergehenden Ansprüche, wobei das Mischorgan (104) eine Strahldüse ist.

**5.** Verfahren zur Durchführung einer kontinuierlichen Reaktion, bei dem man

- ein erstes Fluid höherer Dichte und ein zweites Fluid geringerer Dichte in die rückvermischte Zone (101) eines Reaktors nach einem der vorhergehenden Ansprüche einführt, so dass zumindest das erste Fluid die rückvermischte Zone (101), die Zone begrenzter Rückvermischung (102) und die propfenförmig durchströmte Zone (103) nacheinander durchströmt, und
- das ein Reaktionsprodukt enthaltende erste Fluid am Reaktionsprodukt-Auslass der propfenförmig durchströmten Zone (103) abzieht.

**6.** Verfahren nach Anspruch 5, wobei man nicht umgesetztes zweites Fluid über einen Auslass aus der rückvermischten Zone (101) zumindest teilweise ausführt.

**7.** Verfahren nach Anspruch 5 oder 6, wobei das erste Fluid eine Flüssigkeit und das zweite Fluid ein Gas ist.

**8.** Verfahren nach Anspruch 7, zur Durchführung einer Hochdruckreaktion.

**9.** Verfahren nach Anspruch 7 oder 8, wobei man das Gas in der rückvermischten Zone (101) in der Flüssigkeit dispergiert, die Flüssigkeit die Zone begrenzter Rückvermischung (102) und die propfenförmig durchströmte Zone (103) nacheinander durchströmt, wobei das Volumenverhältnis von dispergiertem Gas zur Flüssigkeit in Strömungsrichtung von der Zone begrenzter Rückvermischung (102) zur propfenförmig durchströmten Zone (103) abnimmt, so dass die propfenförmig durchströmte Zone (103) von der im Wesentlichen einphasigen Flüssigkeit durchströmt wird.

**10.** Verfahren nach einem der Ansprüche 7 bis 9 zur Herstellung einer optisch aktiven Carbonylverbindung durch asymmetrische Hydrierung einer prochiralen, α,β-ungesättigten Carbonylverbindung mit Wasserstoff in Gegenwart eines homogenen Rhodium-Katalysators, der wenigstens einen chiralen Liganden, insbesondere Chiraphos, aufweist.

**11.** Verfahren nach Anspruch 10, wobei das Verfahren in Gegenwart einer Verbindung der Formel (II) durchgeführt wird,

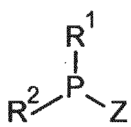

(II)

worin Z in Formel (II) für eine Gruppe $CHR^3R^4$ steht und worin die Variablen $R^1$, $R^2$, $R^3$, $R^4$ unabhängig voneinander und insbesondere gemeinsam die folgenden Bedeutungen aufweisen:

$R^1$, $R^2$: sind gleich oder verschieden und stehen für Phenyl, das unsubstituiert ist oder 1, 2 oder 3 Substituenten trägt, die ausgewählt sind unter Methyl und Methoxy, wobei $R^1$ und $R^2$ jeweils insbesondere für unsubstituiertes Phenyl stehen;
$R^3$ steht für $C_1$- bis $C_4$-Alkyl, insbesondere für Methyl;
$R^4$ steht für $C_1$- bis $C_4$-Alkyl, das eine Gruppe $P(=O)R^{4a}R^{4b}$ trägt und insbesondere eine Gruppe $CH_2$-$P(=O)R^{4a}R^{4b}$ oder $CH(CH_3)$-$P(=O)R^{4a}R^{4b}$ bedeutet;
wobei
$R^{4a}$, $R^{4b}$: gleich oder verschieden sind und für Phenyl stehen, das unsubstituiert ist oder 1, 2 oder 3 Substituenten trägt, die ausgewählt sind unter Methyl und Methoxy, wobei besonders bevorzugt $R^{4a}$ und $R^{4b}$ jeweils für unsubstituiertes Phenyl stehen.

**12.** Verfahren zur Herstellung von optisch aktivem Menthol, bei dem man optisch aktives Citronellal der Formel (III)

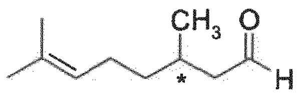

(III)

worin * das Asymmetriezentrum bezeichnet;

nach einem Verfahren der Ansprüche 10 oder 11 durch asymmetrische Hydrierung von Geranial der Formel (Ia-1) oder von Neral der Formel (Ib-1)

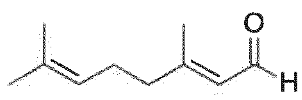

(Ia-1)

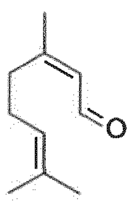

(Ib-1)

oder einer Neral und Geranial enthaltenden Mischung herstellt,

das optisch aktive Citronellal der Formel (III) einer Cyclisierung zu optisch aktivem Isopulegol unterzieht und das optisch aktive Isopulegol zu optisch aktivem Menthol hydriert.

**13.** Verfahren nach einem der Ansprüche 5 oder 6, wobei das erste Fluid eine nicht wasser-mischbare organische Flüssigkeit und das zweite Fluid eine wässrige Füssigkeit ist.

**14.** Verfahren nach Anspruch 13 zur Herstellung eines β-Hydroxyketons, wobei das erste Fluid ein Dialkylketon umfasst und das zweite Fluid eine Formaldehyd-Quelle umfasst.

## Claims

**1.** A reactor for performing a reaction between two immiscible fluids of different density, comprising an interior formed by a cylindrical, vertically oriented elongate shell, a bottom and a cap,

wherein the interior is divided by internals into a backmixed zone (101), a zone of limited backmixing (102) arranged below the backmixed zone (101) and a plug-flow zone (103) which are at least consecutively traversable by one of the fluids,
wherein the backmixed zone (101) comprises at least one inlet and the plug-flow zone (103) comprises an outlet and the backmixed zone (101) comprises at least one mixing apparatus (104) selected from a stirrer, a jet nozzle and means for injecting the fluid of lower density,
a first cylindrical internal element which in the interior extends in the longitudinal direction of the reactor, which delimits the zone of limited backmixing (102) from the plug-flow zone (103) and which comprises a first passage to the backmixed zone (101) and a second passage to the plug-flow zone (103), wherein the first internal element is arranged concentrically to the shell so that the plug-flow zone (103) has an annular horizontal cross section, and wherein the lower edge of the first internal element is arranged at a distance from the bottom of the reactor,
a second internal element which delimits the backmixed zone (101) from the plug-flow zone (103) such that there is no direct fluid connection between the backmixed zone (101) and the plug-flow zone (103), wherein the second internal element extends from the upper edge of the first internal element to the shell, and wherein the second internal element is a horizontally oriented annular plate, and
backmixing-preventing third internal elements in the form of random packings, structured packings or liquid-permeable trays arranged in the zone of limited backmixing (102).

**2.** The reactor according to claim 1, wherein the plug-flow zone (103) is arranged such that the flow through it is turbulent.

**3.** The reactor according to any of the preceding claims, wherein the second internal element divides the interior in the vertical direction into an upper half and a lower half in the ratio of 4:1 to 1:1.

**4.** The reactor according to any of the preceding claims, wherein the mixing apparatus (104) is a jet nozzle.

**5.** A process for performing a continuous reaction, wherein

- a first fluid of higher density and a second fluid of lower density are introduced into the backmixed zone (101) of a reactor according to any of the preceding claims so that at least the first fluid consecutively traverses the backmixed zone (101) , the zone of limited backmixing (102) and the plug-flow zone (103), and
- the first fluid comprising a reaction product is withdrawn at the reaction product outlet of the plug-flow zone (103).

**6.** The process according to claim 5, wherein unconverted second fluid is at least partially discharged from the backmixed zone (101) via an outlet.

**7.** The process according to claim 5 or 6, wherein the first fluid is a liquid and the second fluid is a gas.

**8.** The process according to claim 7 for performing a high-pressure reaction.

**9.** The process according to claim 7 or 8, wherein the gas is dispersed in the liquid in the backmixed zone (101) , the liquid consecutively traverses the zone of limited backmixing (102) and the plug-flow zone (103) , wherein the volume ratio of dispersed gas to the liquid decreases in the flow direction from the zone of limited backmixing (102) to the plug-flow zone (103) so that the plug-flow zone (103) is traversed by the substantially single-phase liquid.

**10.** The process according to any of claims 7 to 9 for producing an optically active carbonyl compound by asymmetric hydrogenation of a prochiral $\alpha,\beta$-unsaturated carbonyl compound with hydrogen in the presence of a homogeneous rhodium catalyst comprising at least one chiral ligand, in particular chiraphos.

**11.** The process according to claim 10, wherein the process is performed in the presence of a compound of formula (II)

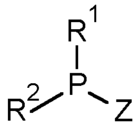

(II)

wherein Z in formula (II) represents a $CHR^3R^4$ group and wherein the variables $R^1$, $R^2$, $R^3$, $R^4$ independently of one another and especially jointly are as follows:

$R^1$, $R^2$: are identical or different and represent phenyl which is unsubstituted or bears 1, 2 or 3 substituents selected from methyl and methoxy, wherein
$R^1$ and $R^2$ each especially represent unsubstituted phenyl;
$R^3$ represents $C_1$- to $C_4$-alkyl, especially methyl;
$R^4$ represents $C_1$- to $C_4$-alkyl bearing a $P(=O)R^{4a}R^{4b}$ group and especially a $CH_2$-$P(=O)R^{4a}R^{4b}$ or $CH(CH_3)$-$P(=O)R^{4a}R^{4b}$ group;
wherein
$R^{4a}$, $R^{4b}$: are identical or different and represent phenyl which is unsubstituted or bears 1, 2 or 3 substituents selected from methyl and methoxy, wherein particularly preferably $R^{4a}$ and $R^{4b}$ each represent unsubstituted phenyl.

**12.** A process for producing optically active menthol in which optically active citronellal of formula (III)

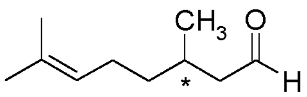

(III)

wherein * denotes the asymmetric center;

is produced by a process according to claim 10 or 11 by asymmetric hydrogenation of geranial of formula (Ia-1) or of neral of formula (Ib-1)

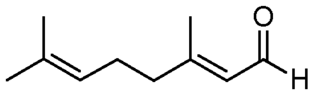

(Ia-1)

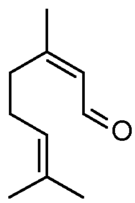

(Ib-1)

or of a mixture comprising neral and geranial,
the optically active citronellal of formula (III) is subjected to a cyclization to afford optically active isopulegol and the optically active isopulegol is hydrogenated to afford optically active menthol.

**13.** The process according to either of claims 5 or 6, wherein the first fluid is a water-immiscible organic liquid and the second fluid is an aqueous liquid.

**14.** The process according to claim 13 for producing a $\beta$-hydroxy ketone, wherein the first fluid comprises a dialkyl ketone and the second fluid comprises a formaldehyde source.

## Revendications

**1.** Réacteur pour la réalisation d'une réaction entre deux fluides non miscibles de densités différentes, comportant

un espace intérieur formé d'un manteau cylindrique allongé orienté verticalement, d'un fond et d'une hotte, l'espace intérieur étant divisé par des éléments intégrés en une zone de remélange (101), une zone de remélange limité (102) située en dessous de la zone de remélange (101) et une zone à écoulement en forme de bouchon (103), qui peuvent être traversées successivement par au moins l'un des fluides,
la zone de remélange (101) présentant au moins une entrée et la zone à écoulement en forme de bouchon (103) présentant une sortie, et la zone de remélange (101) présentant au moins un organe de mélange (104) qui est choisi parmi un agitateur, une buse à jet et des moyens de pressage du fluide de plus faible densité,
un premier élément intégré cylindrique qui s'étend dans l'espace intérieur dans la direction longitudinale du réacteur, qui délimite la zone de remélange limité (102) de la zone à écoulement en forme de bouchon (103) et qui présente un premier passage vers la zone de remélange (101) et un deuxième passage vers la zone à écoulement en forme de bouchon (103), le premier élément intégré étant disposé de manière concentrique par rapport au manteau de manière à ce que la zone à écoulement en forme de bouchon (103) présente une section transversale horizontale en forme d'anneau, et le bord inférieur du premier élément intégré étant agencé à distance du fond du réacteur,
un deuxième élément intégré qui délimite la zone de remélange (101) de la zone à écoulement en forme de bouchon (103) de telle manière qu'aucune liaison fluidique directe n'existe entre la zone de remélange (101) et la zone à écoulement en forme de bouchon (103), le deuxième élément intégré s'étendant du bord supérieur du premier élément intégré vers le manteau, et le deuxième élément intégré étant une tôle annulaire orientée horizontalement, et
des troisièmes éléments intégrés empêchant un remélange sous forme de corps de remplissage, de garnitures structurées ou de fonds perméables aux liquides, qui sont agencés dans la zone de remélange limité (102).

**2.** Réacteur selon la revendication 1, la zone à écoulement en forme de bouchon (103) étant disposée de telle manière qu'elle est traversée de manière turbulente.

**3.** Réacteur selon l'une quelconque des revendications précédentes, le deuxième élément intégré divisant l'espace intérieur dans la direction verticale en une moitié supérieure et une moitié inférieure en un rapport de 4 : 1 à 1 : 1.

**4.** Réacteur selon l'une quelconque des revendications précédentes, l'organe de mélange (104) étant une buse à jet.

**5.** Procédé pour la réalisation d'une réaction en continu, dans lequel

- on introduit un premier fluide de densité plus élevée et un deuxième fluide de densité plus faible dans la zone de remélange (101) d'un réacteur selon l'une quelconque des revendications précédentes, de telle manière qu'au moins le premier fluide traverse successivement la zone de remélange (101), la zone de remélange limité (102) et la zone à écoulement en forme de bouchon (103), et
- on soutire le premier fluide contenant un produit de réaction au niveau de la sortie pour produit de réaction de la zone à écoulement en forme de bouchon (103).

**6.** Procédé selon la revendication 5, dans lequel on évacue au moins partiellement du deuxième fluide qui n'a pas réagi par le biais d'une sortie de la zone de remélange (101).

**7.** Procédé selon la revendication 5 ou 6, le premier fluide étant un liquide et le deuxième fluide étant un gaz.

**8.** Procédé selon la revendication 7, pour la réalisation d'une réaction sous haute pression.

**9.** Procédé selon la revendication 7 ou 8, dans lequel on disperse dans le liquide le gaz dans la zone de remélange (101), le liquide traverse successivement la zone de remélange limité (102) et la zone à écoulement en forme de bouchon (103), le rapport volumique de gaz dispersé sur liquide dans la direction d'écoulement diminuant de la zone de remélange limité (102) à la zone à écoulement en forme de bouchon (103), de telle manière que la zone à écoulement en forme de bouchon (103) soit traversée par le liquide essentiellement monophasique.

**10.** Procédé selon l'une quelconque des revendications 7 à 9 pour la préparation d'un composé carbonylé optiquement actif par hydrogénation asymétrique d'un composé carbonylé prochiral $\alpha,\beta$-insaturé avec de l'hydrogène en présence

d'un catalyseur homogène au rhodium, qui présente au moins un ligand chiral, en particulier le Chiraphos.

**11.** Procédé selon la revendication 10, le procédé étant réalisé en présence d'un composé de formule (II),

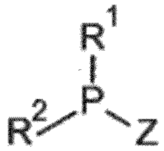

(II)

dans laquelle Z dans la formule (II) représente un groupe $CHR^3R^4$ et dans laquelle les variables $R^1$, $R^2$, R3, $R^4$ présentent indépendamment les unes des autres et en particulier ensemble les significations suivantes :

$R^1$, $R^2$: sont identiques ou différents et représentent phényle, qui est non substitué ou porte 1, 2 ou 3 substituants qui sont choisis parmi méthyle et méthoxy, $R^1$ et $R^2$ représentant à chaque fois en particulier phényle non substitué ;
$R^3$ représente $C_{1-4}$ alkyle, en particulier méthyle ;
$R^4$ représente $C_{1-4}$ alkyle, qui porte un groupe $P(=O)R^{4a}R^{4b}$ et signifie en particulier groupe $CH_2$-$P(=O)R^{4a}R^{4b}$ ou $CH(CH_3)$-$P(=O)R^{4a}R^{4b}$ ;
$R^{4a}$, $R^{4b}$: étant identiques ou différents et représentant phényle, qui est non substitué ou porte 1, 2 ou 3 substituants qui sont choisis parmi méthyle et méthoxy, $R^{4a}$ et $R^{4b}$ représentant à chaque fois de manière particulièrement préférée phényle non substitué.

**12.** Procédé pour la préparation de menthol optiquement actif, dans lequel on prépare du citronellal optiquement actif de formule (III)

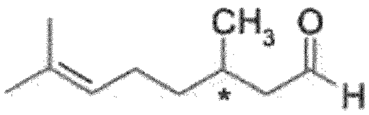

(III)

dans laquelle * désigne le centre d'asymétrie ; par un procédé de la revendication 10 ou 11 par hydrogénation asymétrique de géranial de formule (Ia-1) ou de néral de formule (Ib-1)

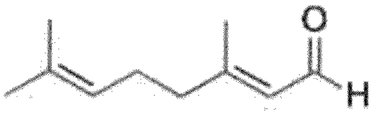

(Ia-1)

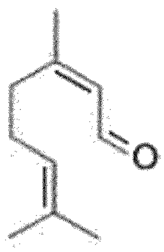

(Ib-1)

ou d'un mélange contenant du néral et du géranial, on soumet le citronellal optiquement actif de formule (III) à une cyclisation pour donner de l'isopulégol optiquement actif et on hydrogène l'isopulégol optiquement actif pour donner du menthol optiquement actif.

**13.** Procédé selon l'une quelconque des revendications 5 et 6, le premier fluide étant un fluide organique non miscible à l'eau et le deuxième fluide étant un liquide aqueux.

**14.** Procédé selon la revendication 13 pour la préparation d'une β-hydroxycétone, le premier fluide comprenant une dialkylcétone et le deuxième fluide comprenant une source de formaldéhyde.

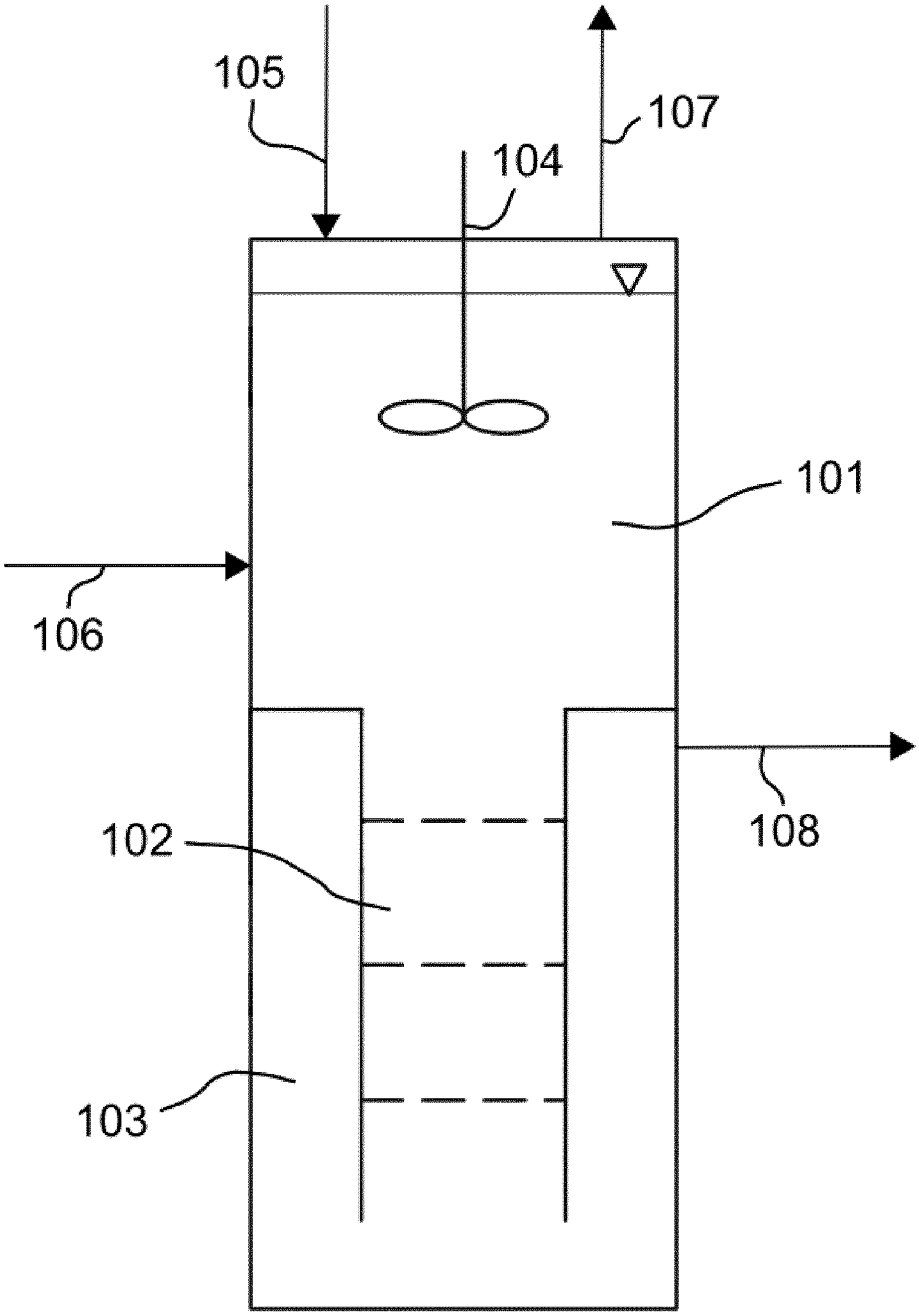


Fig. 1

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente


- WO 2009153123 A
- EP 1338333 A1
- EP 1231198 A1
- DE 102004003468 A1
- US 4000212 A
- WO 2016097242 A1
- US 20180057437 A1
- WO 2006040096 A1
- WO 2008132057 A1


### In der Beschreibung aufgeführte Nicht-Patentliteratur


- Ullmann’s Encyclopedia of Industrial Chemistry. Verlag Chemie, 2008
- Catalytic Asymmetric Synthesis. Wiley-VCh, 2000
- Comprehensive Asymmetric Catalysis. Springer, 2000
- **W. TANG ; X. ZHANG.** *Chem. Rev.,* 2003, vol. 103, 3029-3069